# FASCICULE DE BREVET EUROPEEN

(11) **EP 1 438 330 B1**
(45) Date de publication et mention de la délivrance du brevet: **16.12.2009**
(21) Numéro de dépôt: 02802664.9
(22) Date de dépôt: 17.10.2002
(51) Int. Cl.: C07K 7/08, C12N 15/10, C07K 14/755, A61K 38/10, A61P 7/00

(54) **LEURRES PEPTIDIQUES POUR LA PREPARATION DE MEDICAMENTS DESTINES A LA PREVENTION OU AU TRAITEMENT DES PATHOLOGIES AUTO-IMMUNES, OU DES TROUBLES LIES A L'APPARITION D'ANTICORPS DIRIGES CONTRE DES PROTEINES EXOGENES**
PEPTID-KÖDER ZUR HERSTELLUNG VON MEDIKAMENTEN, DIE FÜR DIE PRÄVENTION ODER BEHANDLUNG VON AUTOIMMUNERKRANKUNGEN BZW. PROBLEMEN, DIE MIT DEM AUFTRETEN VON GEGEN EXOGENE PROTEINE GERICHTETEN ANTIKÖRPERN IN ZUSAMMENHANG STEHEN, BESTIMMT SIND
PEPTIDE DECOYS FOR THE PREPARATION OF MEDICAMENTS THAT ARE INTENDED FOR THE PREVENTION OR TREATMENT OF AUTOIMMUNE PATHOLOGIES OR PROBLEMS LINKED TO THE APPEARANCE OF ANTIBODIES DIRECTED AGAINST EXOGENOUS PROTEINS

(30) Priorité: 17.10.2001 FR 0113360
(43) Date de publication de la demande: 21.07.2004
(73) Titulaire: CENTRE NATIONAL DE LA RECHERCHE SCIENTIFIQUE (CNRS), 75794 Paris Cedex 16 (FR)
(72) Inventeur: GRANIER, Claude, F-34830 Clapiers (FR); VILLARD, Sylvie, 113, rue du Fesquet F-34080 Montpellier (FR)
(74) Mandataire: Grosset-Fournier, Chantal Catherine
(86) Numéro de dépôt international: PCT/FR2002/003557
(87) Numéro de publication internationale: WO 2003/040176

(56) Documents cités:
- WO-A-00/40602
- WO-A-96/02572
- WO-A-98/33920
- WO-A-99/30736
- WO-A-99/46274
- WO-A-99/47932
- US-A- 5 187 155
- NOGAMI KEIJI ET AL: "Identification of a factor VIII peptide, residues 2315-2330, which neutralizes human factor VIII C2 inhibitor alloantibodies: Requirement of Cys2326 and Glu2327 for maximum effect." BRITISH JOURNAL OF HAEMATOLOGY, vol. 107, no. 1, octobre 1999 (1999-10), pages 196-203, XP009008081 ISSN: 0007-1048
- KUWABARA I ET AL: "Mapping of the minimal domain encoding a conformational epitope by lambda phage surface display: factor VIII inhibitor antibodies from haemophilia A patients" JOURNAL OF IMMUNOLOGICAL METHODS, ELSEVIER SCIENCE PUBLISHERS B.V.,AMSTERDAM, NL, vol. 224, no. 1-2, 22 avril 1999 (1999-04-22), pages 89-99, XP004165513 ISSN: 0022-1759
- VOORBERG JAN ET AL: "Phage display technology: A tool to explore the diversity of inhibitors to blood coagulation factor VIII." SEMINARS IN THROMBOSIS AND HEMOSTASIS, vol. 26, no. 2, 2000, pages 143-150, XP008005330 ISSN: 0094-6176
- FERRIERES GAELLE ET AL: "Affinity for the cognate monoclonal antibody of synthetic peptides derived from selection by phage display: Role of sequences flanking the binding motif." EUROPEAN JOURNAL OF BIOCHEMISTRY, vol. 267, no. 6, mars 2000 (2000-03), pages 1819-1829, XP001093880 ISSN: 0014-2956
- TAKAHAASHI I ET AL: "CHANGE OF ANTIGENIC AND NEUTRALIZING SPECIFICITY IN SUBSTITUTIONAL EPITOPE PEPTIDES OF HEMOPHILIA B INHIBITOR" PEPTIDES, ELMSFORD, US, vol. 19, no. 7, 1998, pages 1129-1136, XP001037525 ISSN: 0196-9781
- DOUDNA J A ET AL: "SELECTION OF AN RNA MOLECULE THAT MIMICS A MAJOR AUTOANTIGENIC EPITOPE OF HUMAN INSULIN RECEPTOR" PROCEEDINGS OF THE NATIONAL ACADEMY OF SCIENCES OF USA, NATIONAL ACADEMY OF SCIENCE. WASHINGTON, US, vol. 92, no. 6, 14 mars 1995 (1995-03-14), pages 2355-2359, XP000919062 ISSN: 0027-8424
- SIBILLE P ET AL: "MIMOTOPES OF POLYREACTIVE ANTI-DNA ANTIBODIES IDENTIFIED USING PHAGE-DISPLAY PEPTIDE LIBRARIES" EUROPEAN JOURNAL OF IMMUNOLOGY, WEINHEIM, DE, vol. 27, mai 1997 (1997-05), pages 1221-1228, XP000973084 ISSN: 0014-2980
- MCCONNELL S J ET AL: "Constrained peptide libraries as a tool for finding mimotopes" GENE, ELSEVIER BIOMEDICAL PRESS. AMSTERDAM, NL, vol. 151, no. 1, 30 décembre 1994 (1994-12-30), pages 115-118, XP004042621 ISSN: 0378-1119
- VANHOORELBEKE K ET AL: "SEQUENCE ALIGNMENT BETWEEN VWF AND PEPTIDE INHIBITING THE VWF-COLLAGEN INTERACTION DOES NOT RESULT IN THE IDENTIFICATION OF A COLLAGEN-BINDING SITE IN VWF" THROMBOSIS AND HAEMOSTASIS, STUTTGART, DE, vol. 84, no. 4, octobre 2000 (2000-10), pages 621-625, XP008006899 ISSN: 0340-6245
- SUPHIOGLU C ET AL: "A novel grass pollen allergen mimotope identified by phage display peptide library inhibits allergen-human IgE antibody interaction" FEBS LETTERS, ELSEVIER SCIENCE PUBLISHERS, AMSTERDAM, NL, vol. 502, no. 1-2, 27 juillet 2001 (2001-07-27), pages 46-52, XP004296817 ISSN: 0014-5793
- SEM D S ET AL: "Structural characterization and optimization of antibody-selected phage library mimotopes of an antigen associated with autoimmune recurrent thrombosis." BIOCHEMISTRY. UNITED STATES 17 NOV 1998, vol. 37, no. 46, 17 novembre 1998 (1998-11-17), pages 16069-16081, XP002245692 ISSN: 0006-2960

## Description

L'invention a pour objet des leurres peptidiques d'anticorps pathogènes pour la préparation de médicaments destinés à la prévention ou au traitement des pathologies auto-immunes, ou des troubles liés à l'apparition d'anticorps dirigés contre des protéines exogènes recombinantes ou non utilisées dans le cadre du traitement de ces pathologies auto-immunes ou des pathologies nécessitant l'administration aux patients desdites protéines exogènes.

L'hémophilie A est une pathologie (sévère) due à l'absence ou l'insuffisance de facteur VIII (FVIII) fonctionnel. La capacité de coagulation est restaurée chez les patients par l'administration de concentrés de facteur VIII recombinant ou issu de plasmas.

Il est connu que chez un pourcentage important (environ 15-25%) d'hémophiles A traités par le facteur VIII, un processus d'auto-immunisation aboutit à l'apparition d'anticorps anti-FVIII inhibant la fonction procoagulante du facteur VIII administré, rendant plus complexe encore le traitement des patients. Ces anticorps se lient à plusieurs régions (ou domaines structuraux) de la protéine FVIII, en particulier les domaines C2, A2 et a3-A3-C1.

La réponse anticorps a pour effet d'inhiber l'activité procoagulante du FVIII administré et de compliquer ainsi sérieusement le traitement de ces hémophiles.

Aucune thérapeutique actuelle ne résout complètement ce problème ; la « tolérisation immunologique » par administration de doses massives de facteur VIII a un coût économique important pour le système de santé, ce qui rend nécessaire des recherches pour mettre au point des procédés possédant un rapport efficacité/coût plus favorable.

Il est connu que les anticorps possèdent une structure moléculaire spécialisée, appelée paratope, qui leur permet d'effectuer une fonction biologique importante: la reconnaissance de l'antigène. Le paratope de l'anticorps est capable de reconnaître non seulement une partie limitée (appelée « épitope ») de l'antigène qui a été à l'origine de sa sélection, mais aussi des fragments plus petits de cet antigène, en particulier des peptides correspondant à l'épitope (appelés « peptides épitopiques »), des peptides possédant des modifications de séquence ou de structure chimique par rapport à l'épitope (appelés « variants d'épitopes ») ou encore des molécules n'ayant pas d'homologie de séquence avec l'antigène (appelées « mimotopes »).

La réponse anticorps contre le FVIII est polyclonale et hétérogène dans sa spécificité [Gilles et al, 1993, Blood, 2452-61] mais il a été observé que les régions reconnues par les anticorps inhibiteurs (épitopes) sont restreintes à quelques zones principales de la molécule de facteur VIII. Le domaine A2 et le domaine C2 ont été identifiés initialement par des méthodes d'immunoempreinte utilisant des fragments protéolytiques du FVIII [Fulcher et al., 1985, PNAS USA 82, 7728-32] et confirmés ultérieurement lorsque la séquence nucléotidique du FVIII a été déterminée (Vehar et al., 1984, Nature 312, 337-342 ; Gitschier et al., 1984, Nature 312, 326-336) et des fragments recombinants préparés [Scandella et al., 1988, PNAS 86(4), 1387; Scandella et al., 1989, Blood 74, 1618-26]. Dans un cas particulier, l'épitope d'un anticorps inhibiteur monoclonal murin a pu être restreint à une séquence de 25 acides aminés de la région A2 (résidus 484-508) grâce à l'utilisation astucieuse d'hybrides entre les séquences du facteur VIII humain et porcin [Healey et al., 1995, J. Biol. Chem. 270, 14505-9]. Le clonage d'un anticorps humain à partir de lymphocytes B d'un patient hémophile a permis de montrer que cet anticorps reconnaissait le domaine C2 et inhibait la fonction du FVIII probablement en se liant avec une forte affinité sur le site de liaison du facteur de von Willebrandt [Jacquemin et al., 1998, Blood 92, 496-506]. Enfin, la région a3-A3-C1 semble aussi reconnue par des anticorps inhibiteurs.

Les approches thérapeutiques existant actuellement dans le cadre de la lutte contre les effets de ces anticorps, sont principalement les suivantes :
- stimulation par la desmopressine de la production de FVIII chez les hémophiles modérés [Kesteven et al., 1984, Thromb. Haemost. 52, 50-2];
- activation de la cascade de la coagulation par des concentrés du complexe prothrombique [Lusher et al., 1983, Blood 62, 1135-8];
- administration de FVII recombinant humain activé [Hedner et al., 1993, Transfus. Med Rev. 7, 78-83] ;
- utilisation de FVIII porcin [Hay et al., 1996, Vox Sang 70, 68-9],
- utilisation de concentrés de prothrombine activée (brevet US 4,160,02 (1979) : Method for producing a blood-coagulation-promoting preparation for human blood plasma; brevet US 4,357,321 (1982): Method and composition for treating clotting factor inhibitors ; brevet US 4,663,164 (1987): Aqueous compositions for treating blood clotting inhibitors),
   - utilisation de fragments du facteur VIII (brevet US 4,649,132 (1987): Treatment of factor VIII inhibitors; brevet US 4,769,336 (1987): Treatment of factor VIII inhibitors; brevet US 5,149,637 (1992): Recombinant factor VIII fragments),
   - utilisation d'immun-complexes (brevet US 5,543,145 (1996): Pharmaceutical composition and method for the suppression of factor VIII inhibitor production),
   - utilisation de molécules hybrides facteur VIII humain/porcin (brevet US 5888974: Hybrid human/animal factor VIII).

Une approche conceptuelle des leurres moléculaires, non basée sur des peptides ou dérivés peptidiques, a été récemment décrite dans deux modèles différents de pathologies humaines à médiation humorale: le diabète et la myasthénie gravis. Dans les deux approches, de courtes séquences d'ARN ont été sélectionnées (à partir d'une large banque d'ARN de séquence aléatoire) pour leur capacité à se lier spécifiquement à un anticorps monoclonal représentatif des autoanticorps humains [Lee et al., 1997, Nat. Biotechnol. 15, 41-5 ; Lee et al., 1996, J. Exp. Med. 184, 315-24 ; Hay et al., 1996, susmentionné]. Dans les deux cas, les auteurs parviennent à démontrer que les leurres sélectionnés inhibent la liaison de quelques sérums de patients à leur cible (récepteur de l'insuline et récepteur de l'acétylcholine, respectivement). Cependant, ces observations n'ont pas été validées dans un modèle animal de ces pathologies. Une des faiblesses de cette approche est l'instabilité métabolique des ARN; bien que des ARN modifiés, plus résistants aux nucléases, aient été utilisés dans certaines de ces études, on peut penser que la demi-vie plasmatique de telles molécules soit suffisamment faible pour ôter toute chance au leurre d'agir.

WO 99/30736 concerne en particulier des peptides épitopiques spécifiques de lymphocytes T. Nogami et al concerne des peptides issus du facteur VIII, notamment des peptides mutants ayant la capacité de neutraliser l'activité d'anticorps dirigés contre le facteur VIII humain in vitro. EP321094 concerne en particulier les peptides correspondant aux résidus 351-365 du facteur VIII humain de séquence TDSEMDVVRFDDDNS et 342-356 de séquence EAEDYDDDLTDSGMD et leur utilisation pour bloquer un anticorps inhibiteur du facteur VIII humain. WO 99/74932 concerne une méthode de détection d'une maladie démyélinisante ou de l'encéphalopathie spongiforme en mesurant la présence d'anticorps du type IgA à l'aide de peptides épitopique correspondant à une espèce du genre *Acinetobacter*. Sem et al. Biochemistry, (1998) 37, no46, 16069-16081 concerne en particulier un peptide mimotopique de la cardiolipine se liant à un anticorps anti-cardiolipine d'origine autoimmune. McConnel S.J. et al.Gene, (1994), 151, 115-118 décrit l'introduction d'une contrainte fonctionnelle sur des mimotopes par addition d'un pont cystéine.

La présente invention découle de la mise en évidence par les Inventeurs, que des peptides ou pseudopeptides correspondant à des variants d'épitopes ou à des mimotopes, sont capables de se fixer dans le site d'anticorps anti-FVIII, en empêchant leur liaison au facteur VIII, et en réduisant ainsi l'activité anti-coagulante de cet anticorps.

L'invention a plus particulièrement pour objet l'utilisation susmentionnée de leurres mimotopiques du facteur VIII de formule (I) suivante :

(X₁)ₙ₁-C-Xₐ-C-(X₂)ₙ₂

dans laquelle :
- n1 et n2 indépendamment l'un de l'autre représente 0 ou 1,
- X₁ et X₂ indépendamment l'un de l'autre représente un acide aminé naturel ou non,
- Xₐ représente l'une des séquences suivantes :
   ■ NPSIGDKN SEQ ID NO: 1 ; ou,
   ■ (X₃)n₃-X₄-X₅-G-K-T-X₆-L, séquence dans laquelle
      - n₃ représente 0 ou 1,
      - X₃ représente un acide aminé quelconque,
      - X₄ représente un acide aminé hydrophobe,
      - X₅ représente un acide aminé aromatique,
      - X₆ représente un acide aminé hydrophobe ; ou,
   ■ X₇-X₈-W-X₉-N-R-X₁₀-X₁₁-(X₁₂)ₙ₁₂-(X₁₃)ₙ₁₃, séquence dans laquelle
      - n₁₂ et n₁₃ indépendamment l'un de l'autre représentent 0 ou 1,
      - X₇ à X₁₃ représentent un acide aminé quelconque,
      - le cas échéant W est remplacé par F ou
   ■ (X₁₄)ₙ₁₄-X₁₅-X₁₆-H-X₁₇-W-G-X₁₈-(X₁₉)ₙ₁₉, séquence dans laquelle
      - n₁₄ et n₁₉ indépendamment l'un de l'autre représentent 0 ou 1
      - X₁₄ à X₁₉ représentent un acide aminé quelconque

L'invention concerne plus particulièrement l'utilisation susmentionnée de leurres mimotopiques définis ci-dessus de formule (I) dans laquelle :
- X₁ est choisi parmi Y, E, Q, S, R, A, K, N, ou T,
- X₂ est choisi parmi R, T, H, G, S, L, V, P, F, D, K, A, Q ou I.

A ce titre, l'invention a également plus particulièrement pour objet l'utilisation susmentionnée du leurre mimotopique de formule suivante :
- YCNPSIGDKNCR: SEQ ID NO : 2

L'invention a plus particulièrement pour objet l'utilisation susmentionnée de leurres mimotopiques définis ci-dessus de formule (I), **caractérisés en ce que** :
- X₃ est choisi parmi I, Q, R, T, ou K,
- X₄ est choisi parmi V, T, L, I, M, F, W, ou Y,
- X₅ est choisi parmi F, ou Y,
- X₆ est choisi parmi A, M, Y, P, T, ou V,

A ce titre, l'invention vise plus particulièrement l'utilisation susmentionnée de leurres mimotopiques définis ci-dessus de formules suivantes :
- ECIVYGKTALCT: SEQ ID NO : 3
- QCPTFGKTMLCT: SEQ ID NO : 4
- SCRLFGKTYLCH: SEQ ID NO : 5
- SCTVYGKTPLCG: SEQ ID NO : 6
- RCKTFGKTTLCS: SEQ ID NO : 7
- RCTVYGKTVLCL: SEQ ID NO : 8

L'invention a plus particulièrement pour objet l'utilisation susmentionnée de leurres mimotopiques définis ci-dessus de formule (I), **caractérisés en ce que** :
- X₇ est choisi parmi H, S, T, M, Q, ou G,
- X₈ est choisi parmi T, A, K, R, Q, ou E,
- X₉ est choisi parmi S, A, H, F, V, G, ou T,
- X₁₀ est choisi parmi R, K, L, S, H, T, I, ou A,
- X₁₁ est choisi parmi S, T, V, K, R, Y, M, ou D,
- X₁₂ est choisi parmi S, R, ou L,
- X₁₃ est choisi parmi I, H, ou W.

A ce titre, l'invention a plus particulièrement pour objet l'utilisation susmentionnée de leurres mimotopiques définis ci-dessus de formule (I) choisis parmi ceux de formules suivantes :
- QCHTWSNRRSCL: SEQ ID NO : 9
- SCHAWSNRRTCR: SEQ ID NO : 10
- RCHAWSNRKSCV: SEQ ID NO : 11
- CSKWANRLVSIC: SEQ ID NO : 12
- CSKWHNRSKRHC: SEQ ID NO : 13
- ACTTWSNRSKCP: SEQ ID NO : 14
- ECTRWSNRSRCF: SEQ ID NO : 15
- CMKWSNRSSRWC: SEQIDNO:16
- KCGRWSNRSSCT: SEQ ID NO : 17
- CGRWFNRSDLHC: SEQ ID NO : 18
- ACHEWSNRSTCT: SEQ ID NO : 19
- KCSRWTNRHLCD: SEQ ID NO : 20
- KCTRWTNRHLCS: SEQ ID NO : 21
- KCTRWTNRAHCP: SEQ ID NO : 22
- QCSKWVNRSRCA: SEQ ID NO : 23
- NCQKWTNRRTCL: SEQ ID NO : 24
- QCGRWSNRSYCS: SEQ ID NO : 25
- TCHRWGNRTSCQ: SEQ ID NO : 26
- QCHRWANRISCS: SEQ ID NO: 27
- RCTQWTNRAYCP: SEQ ID NO : 28
- ACTQWSNRHMCG: SEQ ID NO : 29
- TCHPFSNRSTCT: SEQ ID NO : 30

L'invention a encore plus particulièrement pour objet l'utilisation susmentionnée du leurre mimotopique de formule suivante :
- SCHAWSNRRTCR: SEQ ID NO : 10

L'invention a également pour objet l'utilisation susmentionnée des leurres mimotopiques de formule suivante :
- KCEPDDPWPQCI: SEQ ID NO : 31
- ACKRNHRWGACV: SEQ ID NO : 32
- ECGSHAWGRRCK: SEQ ID NO : 33

L'invention concerne plus particulièrement l'utilisation susmentionnée de leurres mimotopiques définis ci-dessus de formule (I), **caractérisés en ce que** :
- X₁₄ représente K,
- X₁₅ représente R, ou G,
- X₁₆ représente N, ou S,
- X₁₇ représente R, ou A,
- X₁₈ représente A, ou R,
- X₁₉ représente R.

A ce titre, l'invention a plus particulièrement pour objet l'utilisation susmentionnée de leurres mimotopiques définis ci-dessus de formule (I) suivante :
- ECGSHAWGRRCK: SEQ ID NO : 35

L'invention concerne également toute composition pharmaceutique **caractérisée en ce qu**'elle comprend des leurres tels que définis ci-dessus, en association avec des véhicules pharmaceutiquement acceptables.

Avantageusement, les compositions pharmaceutiques de l'invention se présentent sous des formes susceptibles d'être administrées par voie parentérale, ou par voie orale.

De préférence la posologie desdites compositions pharmaceutiques est telle qu'elle est comprise entre environ 1 et environ 5 mg/kg/j de composés de formule (I) ou (II) définis ci-dessus.

L'invention concerne également les séquences peptidiques de formule (I) telles que définies ci-dessus.

L'invention concerne également les produits comprenant :
* au moins une séquence peptidique de formule (I) telle que définie ci-dessus
* et au moins un composé choisi parmi le facteur VIII extrait, ou recombinant, ou autres produits dérivés d'origine humaine ou animale,
comme produits de combinaison pour une utilisation simultanée, séparée ou étalée dans le temps, en thérapie de substitution du facteur VIII, dans le cadre du traitement ou de la prophylaxie des accidents hémorragiques chez les patients atteints d'hémophilie A.

L'invention a également pour objet les pseudopeptides dérivés des séquences peptidiques de formule (I) définies ci-dessus, notamment les pseudopeptides obtenus par conversion d'un ou plusieurs, voire la totalité des peptides desdites séquences peptidiques en un acide aminé D, ce qui conduit à un pseudopeptide largement plus résistant à la protéolyse que le peptide initial [Kramer et al., 1998, Protein Eng 11, 941-8].

S'agissant de la synthèse des composés de formule (I) susmentionnés, celle-ci est avantageusement effectuée de la manière suivante.

Dans la procédure d'une synthèse en phase solide d'un polypeptide, le polypeptide souhaité est synthétisé à partir d'un support insoluble telle qu'une résine benzhydryle amine ou chloromethylée (dérivée d'un polystyrène réticulé, et disponible chez des fournisseurs en produits chimiques).

L'acide aminé en C-terminal du polypeptide à synthétiser, dont l'azote α ainsi que les sites réactifs sont protégés par des groupements protecteurs, est fixé à la résine en utilisant des techniques de couplage fort usitées. Le groupement protecteur de la fonction amine est déprotégé (laissant les autres groupements protecteurs, s'ils existent, intacts) de sorte que l'acide aminé suivant dans la séquence (disposant lui aussi de groupements protecteurs adéquats) est fixé et ainsi de suite. Quand le polypeptide est entièrement synthétisé, il est séparé de son support résineux, et tous les groupements protecteurs sont éliminés, puis le polypeptide est récupéré. Exemple de groupements protecteurs appropriés : fluorenyl méthyloxy carbonyl pour les groupements alpha amino, béta ou gamma tertio butyl ester pour les acides aspartique et glutamique, tertio butyl oxy carbonyl pour la lysine et le tryptophane, trityl pour la cystéine, l'asparagine, la glutamine et l'histidine, tertio butyl pour la sérine et la thréonine, 2,2,4,6,7-pentadihydrobenzo furane pour l'arginine.

L'invention sera davantage illustrée à l'aide de la description détaillée qui suit de l'obtention de composés de formule (I) susmentionnés, et de la démonstration de leur capacité à inhiber les anticorps anti-facteur VIII.

### I) MATERIEL ET METHODES

### 1) Techniques pour les leurres peptidiques de type variant d'épitope

### Principe de la technique Spot :

Cette synthèse parallèle de peptides sur membrane Spot (Frank, R. (1992) Spot Synthesis: an easy technique for the positionally addressable, parallel chemical synthesis on a membrane support, tetrahedron. 48, 9217-9232) permet d'obtenir rapidement un grand nombre de peptides différents assemblés simultanément sur un support cellulosique plan. Les peptides sont préparés à une échelle de l'ordre de 50 nmol par spot, sous forme immobilisée. Sur une feuille de cellulose (12-8 cm) sont crées des sites circulaires (spot) d'environ 5 mm de diamètre, chimiquement réactifs qui serviront de point d'ancrage pour fixer l'acide aminé C-terminal du peptide. L'allongement de la chaîne peptidique s'effectue par additions successives des acides aminés du C-terminal vers le N-terminal par réaction d'estérification. Les fonctions aminées des résidus utilisés sont protégées temporairement lors du couplage par un groupement labile (un groupement 9-fluorénylméthyloxycarbonyle ou Fmoc) qui est éliminé en milieu basique. Les chaînes latérales réactives sont bloquées par un groupement stable durant la phase de synthèse, et déprotégées en fin d'assemblage. Un cycle de synthèse se déroule en 4 étapes : premièrement formation d'une liaison peptique entre le NH₂ d'un acide aminé déjà déposé avec le COOH de l'acide aminé suivant, qui est au préalable activé par du N-hydroxybenzotriazole et de la N,N diisopropylcarbodiimide. Deuxièmement, les fonctions NH₂ libres n'ayant pas réagi sont acétylées pour éviter la formation de peptides tronqués. Troisièmement, le groupement Fmoc protecteur du nouvel acide aminé est éliminé en milieu basique par la pipéridine. Quatrièmement, les fonctions aminées ainsi obtenues sont repérées par du bleu de bromophénol. Ce cycle est répété autant de fois que nécessaire. En fin de synthèse, après avoir procédé à la déprotection des chaînes latérales des acides aminés, les peptides qui restent fixés de manière covalente sur la membrane sont analysés par un immunoessai colorimétrique indirect.

### a) Préparation des membranes

La séquence complète du FVIII, à l'exception du domaine B, a été synthétisée sur membrane de cellulose sous forme de pentadécapeptides chevauchant décalés de 2 résidus selon la méthode Spot. Le protocole général a été décrit précédemment par Molina *et al*. (Molina, F., Laune, D., Gougat, C., Pau, B. & Granier, C. (1996) Improved performances of spot multiple peptide synthesis, Pept Res. 9, 151-5). Les membranes sont commercialisées par Abimed (Lagenfeld, Allemagne). Les acides aminés Fmoc et le N-hydroxybenzotriazole ont été commandés chez Novabiochem. Le robot qui effectue les différentes étapes de couplage est un robot ASP222 robot (Abimed). Tous les peptides sont acétylés en leur N-terminal. Une fois les peptides assemblés, les groupes protecteurs des chaînes latérales sont déprotégés par un traitement à l'acide trifluororacétique.

### b) Test immunologique colorimétrique indirect

Après saturation de la membrane dans une solution bloquante, la réactivité des peptides est évaluée par immersion de la membrane dans une solution d'anticorps inhibiteur à tester, utilisée généralement à une concentration de 0.1-1 µg/ml. Après lavages, l'interaction peptide-anticorps est révélée par incubation d'un anticorps anti-Fc marqué à la phosphatase alcaline utilisée au 1:1000 (Sigma), qui en présence de son substrat (BCIP-MTT-MgCl₂ Sigma) donne un précipité bleu au niveau des peptides ayant fixé l'anticorps à tester. Les membranes sont ensuite régénérées pour éliminer le précipité bleu et l'anticorps lié en vue d'autres tests.

### c) Obtention d'épitopes analogues

Une fois l'épitope identifié, une série de peptides analogues est préparée en synthèse Spot en substituant chaque position de la séquence réactive par les 19 acides aminés (à l'exception de la cystéine). L'introduction d'une contrainte par l'ajout d'un pont soufré permet également d'obtenir des épitopes analogues.

### 2) Techniques pour les leurres peptidiques de type « mimotopique »

### Principe de la technique du phage display

Des ligands peptidiques peuvent être facilement obtenus grâce à leur capacité de liaison à un anticorps en utilisant la technique du phage-display (Smith, G. P. (1985) Filamentous fusion phage: novel expression vectors that display cloned antigens on the virion surface, Science. 228, 1315-7), basée sur la présentation de peptides aléatoires à la surface de phages filamenteux. Ces peptides peuvent être exposés soit à la surface de la protéine pIII des phages (nombre maximal 5 copies) soit à la surface de la protéine pVIII (nombre maximal 2700 copies). Ces peptides, de taille très variée (de 6AA jusqu'à 30AA) peuvent être soit linéaires soit contraints par un pont soufré.

### a) Différentes banques de phages peptides utilisées

Pour effectuer le screening des peptides aléatoires, différentes banques ont été utilisées :
* 4 banques peptidiques, où le peptide aléatoire est exprimé à la surface de la pVIII du phage (une 15^{mer} linéaire, une 12^{mer} contrainte, une 17^{mer} semi-contrainte et une 30^{mer} linéaire). Celles-ci, décrites par L.L.C. Bonnycastle (University Burnaby, BC, Canada) (Bonnycastle, L. L., Mehroke, J. S., Rashed, M., Gong, X. & Scott, J. K. (1996) Probing the basis of antibody reactivity with a panel of constrained peptide libraries displayed by filamentous phage, J Mol Biol. 258, 747-62), sont constituées environ de 10¹³ TU/ml et construites à partir du vecteur f88.4, dérivé du phage fd-tet.
* 2 banques peptidiques, où le peptide aléatoire est exprimé à la surface de la pIII du phage (une 9^{mer} linéaire et une 12^{mer} contrainte). Celles-ci sont décrites par le Dr Mazzucchheli L (Mazzucchelli, L., Burritt, J. B., Jesaitis, A. J., Nusrat, A., Liang, T. W., Gewirtz, A. T., Schnell, F. J. & Parkos, C. A. (1999) Cell-specific peptide binding by human neutrophils, Blood. 93, 1738-48), et leur diversité est de l'ordre de 10⁹ TU/ml.

### b) Criblage de la banque de phages (biopanning)

La technique de biopanning est réalisée suivant le protocole décrit par Smith *et al*. susmentionné. Trois tours de sélection et d'amplification sont réalisés, en parallèle pour les banques pIII et pVIII. Pour chaque cycle de sélection, les différents anticorps inhibiteurs, à la concentration de 1-5 µg/ml pour les 2 premiers tours, 0.1-0.5 µg/ml pour le 3^{ème} tour, dans du tampon carbonate (NaHCO₃, 100 mM, pH 8.6), sont adsorbés sur une boîte de Pétri 10x1.5 cm (Falcon 1029) pendant une nuit à 4°C sous agitation. Après 5 lavages de 2 min au TBS-T 0.05% (Tris Buffered Saline : NaCl 1.37 M / KCl 26.8 mM / Tris base 0.5 M-Tween 20 0.05% / pH 7) pour éliminer l'excès d'anticorps, les sites non spécifiques sont saturés pendant 2 h à 37°C avec une solution de TBS-T 0.1%-BSA 3% préalablement filtrée. Après élimination de cette dernière solution par 5 lavages de 2 min au TBS-T 0.05%, 2.10¹¹ TU (unités de transduction) de chaque banque primaire, soit 100 fois la diversité de chaque banque, ou d'éluat de phages issus du tour dé sélection précédent sont incubés une nuit à 4°C sous agitation. Les phages non retenus sont éliminés par 10 lavages de 2 min avec du TBS-T 0.5% suivis de 5 lavages de 2 min avec du TBS-T 0.05%. Les phages retenus sont ensuite élués soit par une élution acide (3 ml d'HCl 0.1 M / glycine / BSA 1 mg/ml préalablement filtrée / pH 2.2, incubation de 30 min à TA sous agitation, puis neutralisation avec 150 µl de Tris-HCl 2 M, pH 9) soit par une élution par compétition avec du FVIII (incubation ON à 4°C). Les différents éluats sont ensuite utilisés pour infecter des cellules *E. Coli* K91 pour amplification. Cette amplification se déroule en 2 étapes : tout d'abord 5 ml d'une suspension bactérienne en phase exponentielle de croissance (absorbance 1.8 à 550 nm) est ajouté à chaque éluat. Après une incubation de 10 min à 37°C sans agitation, 93 ml de milieu Luria-Bertani (LB) avec 0.2 µg/ml de tétracycline (Tc) (banques pVIII) ou 0.75 µg/ml de kanamycine (Ka) (banques pIII) sont ajoutés et incubés 30 min à 37°C à 225 tours par minute (rpm). La concentration est ensuite ajustée à 20 µg/ml pour la Tc et à 75 µg/ml pour la Ka, puis la suspension est incubée à 37°C à 225 rpm pendant la nuit. Ces différentes suspensions bactériennes sont ensuite centrifugées à 4°C à 4000 rpm pendant 25 min, puis à 8000 rpm pendant 12 min, afin de précipiter les bactéries. Le surnageant contenant les phages est repris dans du poly(éthylèneglycol) 8000 / NaCl 2.5 M à raison de 15 ml pour 100 ml de surnageant; la précipitation s'effectue durant la nuit à 4°C. Après centrifugation à 4°C à 8000 rpm pendant 40 min, le culot est repris dans 3 ml de TBS 50 mM / NaCl, et incubé à 37°C pendant 30 min à 150 rpm. Le contenu est transféré dans 3 eppendorfs, centrifugé à 15000 rpm pendant 10 min à 4°C pour éliminer les débris cellulaires, puis transféré dans des ampoules stériles de 1.5 ml et stocké à -80°C.

A chaque étape de sélection, la quantité de phages amplifiés est évaluée par titration, tandis que l'enrichissement en phages affins est évalué par ELISA. Après 3 tours de sélection, 3 étapes de clonage ont permis d'obtenir sur milieu solide (milieu LB-agar-Tc à 20 µg/ml ou Ka à 75 µg/ml) des colonies bactériennes isolées. L'aptitude des différents clones à se lier spécifiquement aux anticorps inhibiteurs est ensuite testée par ELISA.

### c) Elisa Direct: Test de reconnaissance des anticorps inhibiteurs par les phages

Les différents anticorps inhibiteurs utilisés pour le criblage des banques sont immobilisés à 1 µg/ml en tampon NaHCO₃ sur plaque de microtitration (Nunc) durant une nuit à 4°C. Les puits sont saturés avec du PBS-T0.1%-lait 2% pendant 1h30 à 37°C. Les phages issus des différents pannings, dilués au 1/25 en tampon PBS-T0.1%-lait 2% sont ensuite incubés pendant 1h30 à 37°C. Entre chacune de ces incubations, 3 lavages au PBS-T0.1% sont effectués sauf après la saturation. Les phages liés sont détectés à l'aide d'un anticorps anti-M13 couplé à la peroxydase (Amersham Boehringer) dilué au 1:3000, incubé pendant 1 h à 37°C. Après 5 lavages, le substrat de la peroxydase (OPD) est ajouté. La réaction se déroule pendant 30 min à l'obscurité puis une mesure de l'absorbance est effectuée à 450 nm. Après arrêt de la réaction par ajout d'H₂SO₄ 4N, une nouvelle mesure est effectuée à 490 nm.

La spécificité des phages est mise en évidence par l'absence de réactivité avec des anticorps irrelevants.

### d) Détermination de la séquence des oligonucléotides

Le séquençage de l'ADN issu des phages est effectué sur séquenceur automatique (EUROGENTEC). Les amorces utilisées : 5' GTT TTG TCG TCT TTC CAG ACG 3' pour la pIII, et 5' TCG GCA AGC TCT TTT AGG 3' pour la pVIII, sont localisées en aval de l'insert peptidique.

### 3) Capacité des peptides variants épitopiques ou mimotopiques sous forme soluble à inhiber leur anticorps inhibiteurs in vitro and ex vivo

### a) Synthèse de peptides solubles

Les différents épitopes identifiés en Spot ou les mimotopes déterminés par la technique du phage display sont ensuite synthétisés sous forme de peptides synthétiques solubles grâce à un synthétiseur Abimed AMS422 basé sur de la chimie Fmoc en phase soluble (Gausepohl, H., Boulin, C., Kraft, M. & Frank, R. W. (1992) Automated multiple peptide synthesis, Pept Res. 5, 315-20). Les peptides sont déprotégés et clivés de la résine par un traitement avec de l'acide trifluoroacétique en présence de scavengers appropriés. Les peptides sont alors lyophilisés, et leur pureté est évaluée en HPLC. Si nécessaire, les peptides sont purifiés en vue d'obtenir plus de 90% d'homogénéité en HPLC.

### b) Capacité neutralisante des leurres in vitro

Le FVIII est immobilisé sur la plaque ON à 4°C. En parallèle, l'anticorps inhibiteur est préincubé avec son/ses peptides épitopiques en gamme ON à 4°C dans du PBS-T0.1% -lait 2%. Après 1 h de saturation avec du PBS-T0.1% -lait 2%, les mélanges Ac-peptide sont incubés avec le FVIII immobilisé pendant 2 h à 37°C. Entre chacune de ces incubations, 3 lavages au PBS-T0.1% sont effectués sauf après la saturation. La détection du complexe FVIII-Ac est obtenue par un anti-IgG de souris ou humain couplé à la peroxydase dilué au 1:3000 incubé pendant 1 h à 37°C. Après 5 lavages de PBS-T0.1%, le substrat de la peroxydase (OPD) est ajouté. La réaction se déroule pendant 30 min à l'obscurité puis une mesure de l'absorbance est effectuée à 450 nm. Après arrêt de la réaction par ajout de H₂SO₄ 4N, une nouvelle mesure est effectuée à 490nm.

### c) Capacité neutralisante des leurres dans un test fonctionnel (Test Bethesda)

Ce test fonctionnel (Kasper, C. K. & Pool, J. G. (1975) Letter: Measurement of mild factor VIII inhibitors in Bethesda units, Thromb Diath Haemorrh. 34, 875-6) consiste à mesurer le temps nécessaire à un mélange composé des différents facteurs de la coagulation (FIXa, FVIII, phospholipides, calcium...) pour coaguler complètement. L'effet inhibiteur d'un anticorps se caractérisera dans un tel test par un allongement du temps du coagulation, la capacité inhibitrice de l'anticorps sera d'autant plus élevée que l'allongement du temps de coagulation sera importante. Le caractère inhibiteur d'un anticorps est exprimé en unité Bethesda, ce qui correspond à la concentration réciproque d'anticorps pour neutraliser 50% de l'activité coagulante du FVIII. Par contre, l'effet neutralisant des peptides se traduira par un retour au temps de coagulation initial obtenu en absence d'inhibiteur.

Le test fonctionnel suivi découle du test décrit par Kasper susmentionné. Les anticorps monoclonaux anti-FVIII sont dilués dans un tampon imidazole 0.05 M, pH 7.3. Un volume égal de cette préparation (250µl) avec du plasma normal tamponné dans de l'imidazole (0.1 M, pH 7.4) sont mélangés et incubés pendant 2 h à 37°C. De manière similaire, du plasma normal tamponné dans de l'imidazole est mélangé à un anticorps non-spécifique lequel constitue le contrôle. L'activité inhibitrice d'un anticorps est lue à partir d'un graphe semi-logarithmique représentant la corrélation entre l'activité résiduelle du FVIII et l'activité inhibitrice.

Pour mettre en évidence la capacité des peptides à neutraliser les anticorps inhibiteurs, l'anticorps inhibiteur utilisé à une concentration de sorte à obtenir 50% de FVIII résiduel, est préincubé ON à 4°C dans du tampon imidazole avec des concentrations croissantes en peptides. Ce mélange est ensuite traité comme ci-dessus.

### II) DESCRIPTIF DETAILLE

### 1) Choix des anticorps :

Il a été utilisé :
- un anticorps monoclonal de souris, ci-après dénommé ESH8, qui présente une très grande activité de neutralisation de l'activité procoagulante du FVIII (6300 Besthesda Unit/mg [Scandella et al., 1995, Blood 86, 1811-9]). Cet anticorps est un bon représentant des anticorps humains se liant au domaine C2 du facteur VIII,
- un anticorps monoclonal humain 2C 11 (Jacquemin et al., 1998, susmentionné),
- plusieurs anticorps monoclonaux de souris, ci-après dénommés F7B4, F29F11, F14A12, F18B1, F19C2 et F21C1 qui représentent un modèle des anticorps humains se liant à la région acide a₁ du facteur VIII pour les 3 premiers, et la région acide a₃ pour les derniers.

### 2) Procédés pour obtenir des leurres :

Il a été obtenu des leurres peptidiques de type "variants d'épitopes", en utilisant la technique Spot pour introduire des variations de la séquence en acides aminés de l'épitope. Les peptides variants d'épitope conservent la réactivité avec l'anticorps ou peuvent montrer une réactivité plus forte que l'épitope. Par exemple, une telle approche concernant l'épitope D³⁵⁶VVRF³⁶⁰ de l'anticorps anti-FVIII F7B4 a montré que la F³⁶⁰ pouvait être substituée sans perte de réactivité par les 2 autres acides aminés aromatiques W et Y. Dans un autre exemple, la présentation de l'épitope sous forme cyclique a été évaluée avec le peptide CDDDLTDSEMDVVRFDC et l'importance des charges négatives a été étudiée avec le peptide AAALTDSEMDVVRFA. Tous ces leurres de type « variants d'épitopes » ont été obtenus par synthèse peptidique conventionnelle et leur activité de liaison à l'anticorps anti-facteur VIII mesurée.

Il a été obtenu des leurres peptidiques de type « mimotopes » par la technique des banques de phages recombinants. L'approche de sélection de peptides à partir de banques aléatoires de séquences a été décrite [Smith et al., 1993, Methods Enzymol. 217, 228-257]. Des peptides portés par des phages filamenteux à la surface de leur protéine d'enveloppe (pVIII ou pIII) peuvent être utilisés: par exemple, une banque codant pour des peptides de 15 acides aminés, et une autre codant pour des peptides de 12 acides aminés, contraints structuralement par la présence constante de deux résidus de cystéine. Les conditions de sélection des phages utilisées sont celles décrites (notamment par [Ferrieres et al., 2000, Eur. J. Biochem. 267, 1819-1829]). L'élution des phages spécifiquement capturés par l'anticorps est effectuée soit par traitement acide soit par déplacement avec un excès d'antigène. La séquence en acides aminés de chaque peptide sélectionné peut être déterminée comme décrit notamment par Ferrières et al. Par exemple, des peptides mimotopes de l'anticorps ESH8, ainsi qu'avec l'anticorps humain 2C11 ont pu être obtenus. Suivant la condition d'élution utilisée, les motifs consensus obtenus varient. Par élution acide, deux groupes de séquences, toutes étant issues de la banque contrainte de 12 acides aminés, sont obtenus : le premier de type mimotopique comprend une seule séquence YCNPSIGDKNCR, séquence représentée par 3 clones, le second groupe de type épitopique se distingue par un motif consensus: X-C-X- hydrophobe-aromatique (Y/F)-G-K-T-X-L-C-X (où X représente n'importe quel acide aminé), qui présente une homologie de séquence avec le domaine C2 du facteur VIII. Ce motif consensus est retrouvé dans 6 différentes séquences: ECIVYGKTALCT (représentée 1 fois), QCQTFGKTMLCT (4 fois), SCRLFGKTYLCH (1 fois), SCTVYGKTPLCG (11 fois), RCKTFGKTTLCS (1 fois) et RCTVYGKTVLCL (1 fois). Tandis qu'avec l'élution avec l'antigène, une seule séquence est obtenue et elle provient de la banque linéaire de 15 acides aminés KPGEVPRHRVTDFDR, représentée 4 fois.

Pour vérifier que ces séquences prises en dehors du contexte du phage soient toujours réactives avec l'anticorps ESH8, celles-ci ont été synthétisées sur membrane par la technique Spot. Il s'avère que toutes ont gardé leur réactivité. Ensuite, pour déterminer les résidus clés de la liaison ESH8/peptide, chaque acide aminé du peptide a été substitué par les 19 autres acides aminés (à l'exception de la cystéine) pour déterminer 1) quels étaient les résidus critiques à la liaison ESH8/peptide 2) les éventuelles substitutions tolérables ce qui permet de dégager une caractéristique commune (exemple caractère hydrophobe ou aromatique, présence d'une longue chaîne latérale). Les résidus comme la glycine (G), la lysine (K), la thréonine (T) et la leucine (L) ne tolèrent aucune substitution, par contre quant au résidu hydrophobe, les éléments les plus réactifs sont valine (V), isoleucine (I) et leucine (L), mais acceptent tout à fait des résidus tels que la thréonine (T), la méthionine (M), la phénylalanine (F), le tryptophane (W), et la tyrosine (Y). Quant au résidu aromatique, cette position tolère principalement la phénylalanine (F), et la tyrosine (Y) avec une légère acceptation du tryptophane (W).

Une fois ces séquences identifiées, un leurre est obtenu par synthèse peptidique conventionnelle.

### 3) Procédés pour déterminer l'activité bloquante des leurres :

L'activité bloquante des leurres peut être vérifiée *in vitro* en test ELISA. Le format utilisé permet de mettre en évidence l'inhibition de la liaison de l'anticorps inhibiteur au facteur VIII par le leurre introduit en concentration croissante. La capacité neutralisante du leurre peut être confirmée dans un test fonctionnel comme le Test Bethesda (méthode de Kasper, [Kasper et al., 1975, Thromb. Diath. Haemorrh. 34, 875-6]).

Les 7 peptides mimotopes solubles plus particulièrement étudiés sont les suivants :
- 103: KCGRWSNRSSCT
- 104: ECGSHAWGRRCK
- 105: CSKWHNRSKRHC
- 106: CMKWSNRSSRWC
- 107: SCHAWSNRRTCR
- 108: QCHTWSNRRSCL
- 109: RCHAWSNRKSCV

Les Inventeurs ont étudié la capacité de ces peptides solubles à inhiber la liaison de l'anticorps 2C11 au facteur VIII dans un test ELISA *in vitro* de la manière suivante : immobilisation du FVIII, pré-incubation de 2C11 avec les peptides solubles en gamme (200µM, 20µM, 2µM, 200nM, 20nM, 2nM) toute la nuit à 4°C, puis incubation de ce prémélange sur le FVIII, révélation ensuite de l'anticorps 2C11. Les résultats obtenus sont indiqués ci-après.

| % d'inhibition | 103 | 104 | 105 | 106 | 107 | 108 | 109 |
|---|---|---|---|---|---|---|---|
| 200 | 62,73 | 63,71 | 75,58 | 36,56 | 90,48 | 60,69 | 75,05 |
| 20 | 28,26 | 30,68 | 45,04 | 21,99 | 80,04 | 25,99 | 37,48 |
| 2 | 3,47 | 13,44 | 10,27 | 0,00 | 37,33 | 15,79 | 12,46 |
| 0,02 | 0 | 0 | 0 | 0 | 0 | 0 | 0 |
| 0,002 | 0 | 0 | 0 | 0 | 0 | 0 | 0 |

Tous les peptides testés inhibent la liaison Ac 2C11/FVIII, le peptide 107 se révélant être le plus efficace.

### 4) Capacité du peptide 107 (SCHAWSNRRTCR) à neutraliser l'anticorps 2C11 dans des analyses fonctionnelles et in vivo.

Comme le peptide 107 s'est avéré un peptide relativement efficace à inhiber la liaison FVIII-anticorps 2C11, les Inventeurs se sont intéressés plus en détails à sa capacité à neutraliser l'activité inhibitrice de 2C 11.

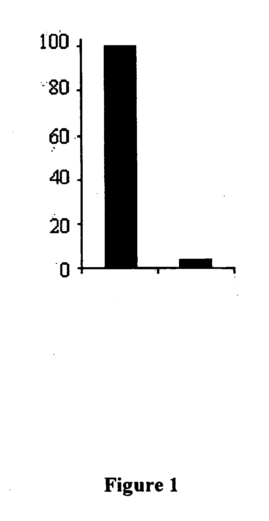

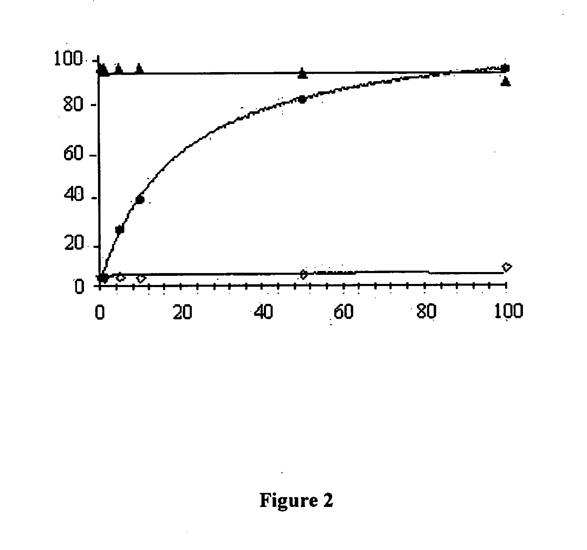

Dans un premier temps, les Inventeurs ont évalué dans un essai fonctionnel de coagulation (test Bethesda) la capacité du peptide 107 à restaurer l'activité procoagulante de FVIII en présence de 2C11. La **Figure 2** dépeint la neutralisation dose-dépendante de l'activité inhibitrice de 2C 11 par le peptide 107. A une concentration en 2C11 de 3,5 nM, concentration à laquelle 98% de l'activité du FVIII est inhibée (**Figure 1**), la neutralisation totale de son effet inhibiteur est atteinte avec une concentration en peptide 107 de l'ordre de 100 µM, l'IC₅₀ étant égale à 19 µM. Un peptide contrôle utilisé dans une gamme de concentration identique ne présente aucun effet neutralisant (**Figure 2**). Le peptide 107 incubé avec du FVIII seul ne modifie nullement l'activité procoagulante du FVIII (**Figure 2**). Les peptides (103, 104, 105, 106, 108, et 109) peuvent également neutraliser efficacement l'activité inhibitrice de 2C 11. Cependant, des concentrations plus élevées en peptide sont nécessaires pour atteindre une efficacité semblable à celle du peptide 107 (données non montrées).

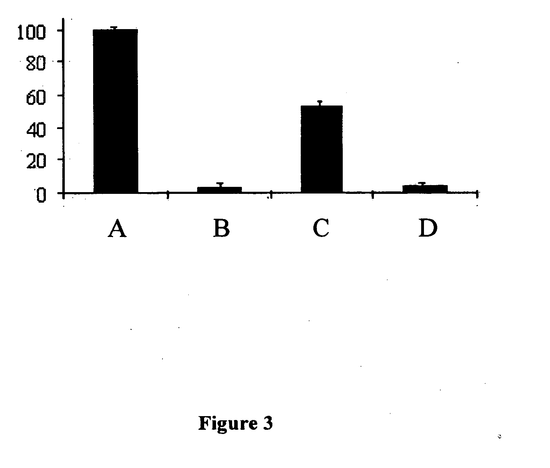

Dans un deuxième temps, les propriétés de neutralisation du peptide 107 ont été étudiées *in vivo*, en utilisant un modèle de souris déficientes en FVIII. Une étude préliminaire a permis de définir qu'une administration intraveineuse de 0,5 UI de FVIII recombinant humain à de telles souris permettait d'obtenir un taux plasmatique en FVIII de l'ordre de 0,3 IU/ml FVIII, taux stable pour au moins une heure. Ceci déterminé, de l'anticorps 2C11 (à 16,7 nM) incubé seul, en présence du peptide 107, ou d'un peptide contrôle (650 µM et 700 µM, respectivement) a été injecté dans la veine de la queue de trois groupes de souris respectivement. Trente minutes plus tard, le taux plasmatique en FVIII de ces souris a été reconstitué par l'injection des 0,5 UI de FVIII humain et après quinze minutes l'activité procoagulante plasmatique a été mesurée. La **Figure 3** prouve que l'administration de 2C 11 seul a complètement inhibé l'activité procoagulante du FVIII injecté, alors que les souris qui ont reçu l'anticorps 2C11 en présence du peptide 107 maintiennent 52% de l'activité procoagulante du FVIII. L'activité neutralisante du peptide 107 est bien spécifique puisqu'un peptide contrôle ne présente aucun effet neutralisant.

Dès lors, ces données indiquent que la capacité neutralisante du peptide 107 sur l'inhibition de l'anticorps 2C11 vis à vis du FVIII, suggérée par des expériences *in vitro,* est également vérifiée dans des conditions *in v*ivo, qui ressemblent à l'hémophilie A.

Dans un troisième temps, les Inventeurs ont regardé si les propriétés neutralisantes du peptide 107 vis à vis de 2C11 pouvaient également s'appliquer à d'autres sérums de patients hémophiles avec inhibiteurs. Une étude préliminaire révèle que sur 12 sérums de patients hémophiles avec inhibiteurs, 2 d'entre eux réagissent avec le peptide 107 indiquant ainsi que des inhibiteurs du FVIII semblables à 2C11 sont produits par d'autres patients présentant l'hémophilie A.

### Figure 1

La figure 1 représente l'effet de l'anticorps 2C11 sur l'activité du facteur VIII évaluée par le test Bethesda. Du facteur VIII a été incubé en présence de 3,5 nM d'anticorps 2C11 durant 2h à 37°C, l'activité résiduelle du facteur VIII a alors été mesurée avec un coagulomètre, en une étape.

Le pourcentage d'activité résiduelle du facteur VIII est représenté en ordonnée. La barre A représente l'activité du facteur VIII seul (100%), la barre B représente l'activité facteur VIII en présence de l'anticorps 2C11 à la concentration de 3,5 nM.

En présence de 3,5 nM de 2C11 l'activité du facteur VIII est inhibée quasi complètement.

### Figure 2

La figure 2 représente l'évolution de l'activité du facteur VIII, évaluée par le test Bethesda, en présence de 3,5 nM d'anticorps 2C11 en fonction de la concentration en peptide. L'anticorps 2C11 a été incubé une nuit à 4°C en présence d'un peptide contrôle ou du peptide 107 (SCHAWSNRRTCR) en concentration variable. Le facteur VIII a alors été incubé 2h à 37°C avec ces mélanges peptide-anticorps ou avec du peptide 107 seul puis son activité résiduelle a été mesurée avec un coagulomètre, en une étape.

Le pourcentage d'activité résiduelle du facteur VIII est représenté en ordonnée, la concentration en peptide est représentée en abscisse (µM). Les triangles noirs représentent l'évolution de l'activité résiduelle du facteur VIII en présence de concentrations croissantes du peptide 107 seul, les points noirs représentent l'évolution de l'activité résiduelle du facteur VIII en présence du mélange entre des concentrations croissantes du peptide 107 et l'anticorps 2C11, les losanges blancs représentent l'évolution de l'activité résiduelle du facteur VIII en présence du mélange entre des concentrations croissantes du peptide contrôle et l'anticorps 2C11.

Le peptide 107 permet de neutraliser l'effet de l'anticorps 2C 11.

### Figure 3

La figure 3 représente l'effet de l'injection de l'anticorps 2C11 en absence ou en présence du peptide 107 ou d'un peptide contrôle sur la coagulation sanguine de souris déficientes en facteur VIII et auxquelles on administre du facteur VIII humain.

L'anticorps 2C11 (16,5 nM) ou un mélange contenant l'anticorps 2C11 (16,5 nM) et le peptide 107 (650 µM) ou un peptide contrôle (700 µM) a été injecté dans la veine de la queue d'une souris « *knock out* », déficiente en facteur VIII, à raison de 2,5 µg d'anticorps 2C11, 1 mg de peptide 107 et 1 mg de peptide contrôle. Après 30 minutes, 0,5 UI de facteur VIII humain a été injecté et l'activité procoagulante plasmatique a alors été mesurée par un test chromogénique 15 minutes plus tard.

Le pourcentage d'activité résiduelle du facteur VIII est représenté en ordonnée. La barre A représente l'effet de l'injection de facteur VIII sans injection préalable d'anticorps (100% d'activité résiduelle). La barre B représente l'effet de l'injection de l'anticorps 2C11 seul préalablement à l'injection de facteur VIII. La barre C représente l'effet de l'injection d'un mélange entre l'anticorps 2C11 et le peptide 107 préalablement à l'injection de facteur VIII. La barre D représente l'effet de l'injection d'un mélange entre l'anticorps 2C11 et un peptide contrôle préalablement à l'injection de facteur VIII.

Le peptide 107 neutralise spécifiquement l'effet de l'anticorps 2C11 *in vivo*.

## Revendications

1. Utilisation de leurres mimotopiques du facteur VIII de formule (I) suivante :
(X₁)ₙ₁-C-Xₐ-C-(X₂)ₙ₂
dans laquelle :
- n1 et n2 indépendamment l'un de l'autre représente 0 ou 1,
- X₁ et X₂ indépendamment l'un de l'autre représente un acide aminé naturel ou non,
- Xₐ représente l'une des séquences suivantes :
■ NPSIGDKN SEQ ID NO : 1 ; ou,
■ (X₃)ₙ₃-X₄-X₅-G-K-T-X₆-L, séquence dans laquelle
- n₃ représente 0 ou 1,
- X₃ représente un acide aminé quelconque,
- X₄ représente un acide aminé hydrophobe,
- X₅ représente un acide aminé aromatique,
- X₆ représente un acide aminé hydrophobe ; ou,
■ X₇-X₈-W-X₉-N-R-X₁₀-X₁₁(X₁₂)ₙ₁₂-(X₁₃)ₙ₁₃, séquence dans laquelle
- n₁₂ et n₁₃ indépendamment l'un de l'autre représentent 0 ou 1,
- X₇ à X₁₃ représentent un acide aminé quelconque,
- le cas échéant W est remplacé par F ou
■ (X₁₄)ₙ₁₄-X₁₅-X₁₆-H-X₁₇-W-G-X₁₈-(X₁₉)ₙ₁₉, séquence dans laquelle
- n₁₄ et n₁₉ indépendamment l'un de l'autre représentent 0 ou 1
- X₁₄ à X₁₉ représentent un acide aminé quelconque.
pour la préparation de médicaments destinés à la prévention ou au traitement des troubles liés à l'apparition d'anticorps contre le facteur VIII endogène, ou contre le facteur VIII exogène ou dérivés, recombinants ou non, administrés dans le cadre de l'hémophilie A.

2. Utilisation selon la revendication 1, de leurres mimotopiques de formule (I) dans laquelle :
- X₁ est choisi parmi Y, E, Q, S, R, A, K, N, ou T,
- X₂ est choisi parmi R, T, H, G, S, L, V, P, F, D, K, A, Q ou I.

3. Utilisation selon la revendication 1 de leurres mimotopiques de formule (I) dans laquelle :
- Xₐ représente YCNPSIGDKNCR SEQ ID NO : 2 ;

4. Utilisation selon la revendication 1, de leurres mimotopiques pour lesquels Xₐ représente la séquence (X₃)n₃-X₄-X₅-G-K-T-X₆-L **caractérisée en ce que:**
- X₃ est choisi parmi I, Q, R, T, ou K,
- X₄ est choisi parmi V, T, L, I, M, F, W, ou Y,
- X₅ est choisi parmi F, ou Y,
- X₆ est choisi parmi A, M, Y, P, T, ou V,

5. Utilisation selon la revendication 1 ou 4, de leurres mimotopiques de formules suivantes :
ECIVYGKTALCT SEQ ID NO : 3
QCPTFGKTMLCT SEQ ID NO : 4
SCRLFGKTYLCH SEQ ID NO : 5
SCTVYGKTPLCG SEQ ID NO : 6
RCKTFGKTTLCS SEQ ID NO : 7
RCTVYGKTVLCL SEQ ID NO : 8

6. Utilisation selon la revendication 1, de leurres mimotopiques pour lesquels Xₐ représente la séquence :
X₇-X₈-W-X₉-N-R-X₁₀-X₁₁-(X₁₂)ₙ₁₂-(X₁₃)ₙ₁₃
**caractérisée en ce que :**
- X₇ est choisi parmi H, S, T, M, Q, ou G,
- X₈ est choisi parmi T, A, K, R, Q, ou E,
- X₉ est choisi parmi S, A, H, F, V, G, ou T,
- X₁₀ est choisi parmi R, K, L, S, H, T, I, ou A,
- X₁₁ est choisi parmi S, T, V, K, R, Y, M, ou D,
- X₁₂ est choisi parmi S, R, ou L,
- X₁₃ est choisi parmi I, H, ou W.

7. Utilisation selon la revendication 1 ou 6, de leurres mimotopiques choisis parmi ceux des formules suivantes :
QCHTWSNRRSCL SEQ ID NO: 9
SCHAWSNRRTCR SEQ ID NO : 10
RCHAWSNRKSCV SEQ ID NO : 11
CSKWANRLVSIC SEQ ID NO : 12
CSKWHNRSKRHC SEQ ID NO : 13
ACTTWSNRSKCP SEQ ID NO : 14
ECTRWSNRSRCF SEQ ID NO : 15
CMKWSNRSSRWC SEQ ID NO : 16
KCGRWSNRSSCT SEQ ID NO : 17
CGRWFNRSDLHC SEQIDNO: 18
ACHEWSNRSTCT SEQ ID NO : 19
KCSRWTNRHLCD SEQ ID NO : 20
KCTRWTNRHLCS SEQ ID NO : 21
KCTRWTNRAHCP SEQ ID NO : 22
QCSKWVNRSRCA SEQ ID NO : 23
NCQKWTNRRTCL SEQ ID NO : 24
QCGRWSNRSYCS SEQ ID NO : 25
TCHRWGNRTSCQ SEQ ID NO : 26
QCHRWANRISCS SEQ ID NO : 27
RCTQWTNRAYCP SEQ ID NO : 28
ACTQWSNRHMCG SEQ ID NO : 29
TCHPFSNRSTCT SEQ ID NO : 30

8. Utilisation selon la revendication 7, du leurre mimotopique de formule suivante :
SCHAWSNRRTCR SEQ ID NO : 10

9. Utilisation selon la revendication 1 ou 2, des leurres mimotopiques de formule suivante :
KCEPDDPWPQCI SEQ ID NO : 31
ACKRNHRWGACV SEQ ID NO : 32
ECGSHAWGRRCK SEQ ID NO : 33

10. Utilisation selon la revendication 1, de leurres mimotopiques pour lesquels Xₐ représente la séquence (X₁₄)ₙ₁₄-X₁₅-X₁₆-H-X₁₇-W-G-X₁₈-(X₁₉)ₙ₁₉,
**caractérisée en ce que** :
- X₁₄ représente K,
- X₁₅ représente R, ou G,
- X₁₆ représente N, ou S,
- X₁₇ représente R, ou A,
- X₁₈ représente A, ou R,
- X₁₉ représente R.

11. Utilisation selon la revendication 1, de leurres mimotopiques pour lesquels Xₐ représente la séquence (X₁₄)ₙ₁₄-X₁₅-X₁₆-H-X₁₇-W-G-X₁₈-(X₁₉)ₙ₁₉, et possède la formule suivante :
ECGSHAWGRRCK SEQ ID NO : 35

12. Composition pharmaceutique **caractérisée en ce qu'**elle comprend des leurres tels que définis dans l'une des revendications 1 à 11, en association avec des véhicules pharmaceutiquement acceptables.

13. Peptides de formule (I) telles que définies dans l'une des revendications 1 à 11.

14. Produits comprenant :
* au moins un peptide de formule (I) selon la revendication 13,
* et au moins un composé choisi parmi le facteur VIII extrait, ou recombinant, ou autres produits dérivés d'origine humaine ou animale,
comme produits de combinaison pour une utilisation simultanée, séparée ou étalée dans le temps, en thérapie de substitution du facteur VIII, dans le cadre du traitement ou de la prophylaxie des accidents hémorragiques chez les patients atteints d'hémophilie A.

## Claims

1. Use of mimotope decoys of factor VIII of formula (I)
(X₁)ₙ₁-C-Xₐ-C-(X₂)ₙ₂
wherein
- n1 and n2 independently of each other represent 0 or 1,
- X₁ et X₂ independently of each other represent a natural or not natural amino acid,
- Xₐ represents one of the following sequences :
* NPSIGDKN SEQ ID NO : 1 or
* (X₃)ₙ₃-X₄-X₅-G-K-T-X₆-L wherein
- n₃ represents 0 or 1,
- X₃ represents any amino acid,
- X₄ represents a hydrophobic amino acid,
- X₅ represents any aromatic amino acid,
- X₆ represents a hydrophobic amino acid ; or
* X₇-X₈-W-X₉-N-R-X₁₀-X₁₁-(X₁₂)ₙ₁₂-(X₁₃)ₙ₁₃ wherein
- n₁₂ and n₁₃ each independently represent 0 or 1,
- X₇ to X₁₃ represent any amino acid,
- optionally W is replaced by F or
- hydrophobic amino acid ; or
* (X₁₄)ₙ₁₄-X₁₅-X₁₆-H-X₁₇-W-G-X₁₈-(X₁₉)ₙ₁₉ sequence wherein
- n₁₄ and n₁₉ independently from each other represent 0 or 1,
- X₁₄ to X₁₉ represent any amino acid,
for the preparation of a drug for the prevention or the treatment of disorders linked to the appearance of antibodies against endogenous factor VIII, exogenous factor VIII derivatives thereof, recombinant or non-recombinant versions thereof, administered in case of hemophilia A.

2. The use according to claim 1 of mimotope decoys of formula (I) wherein:
- X₁ is Y,E,Q,S,R,A, K, N, or T; and
- X₂ is R, T, H, G, S, L, V, P, F, D, K, A, Q or I.

3. The use according to claim 1 of mimotope decoys of formula (I) wherein:
- X₈ represents YCNPSIGDKNCRSEQ ID NO : 2

4. The use according to claim 1 of mimotope decoys wherein X₈ represents the sequence (X₃)ₙ₃-X₄-X₅-G-K-T-X₆-L **characterized in that**
- X₃ is selected from I, Q, R, T, or K,
- X₄ is selected from V, T, L, I, M, F, W, or Y,
- X₅ is selected from F, or Y,
- X₆ is selected from A, M, Y, P, T, or V

5. The use according to claim 1 or 4 of mimotope decoys of the following formulas
ECIVYGKTALCT SEQ ID NO : 3
QCPTFGKTMLCT SEQ ID NO : 4
SCRLFGKTYLCH SEQ ID NO : 5
SCTVYGKTPLCG SEQ ID NO : 6
RCKTFGKTTLCS SEQ ID NO : 7
RCTVYGKTVLCL SEQ ID NO : 8

6. The use according to claim 1 of mimotope decoys wherein X₈ represents the sequence:
X₇-X₈-W-X₉-N-R-X₁₀-X₁₁-(X₁₂)ₙ₁₂-(X₁₃)ₙ₁₃
**characterized in that**:
- X₇ is H, S, T, M, Q, or G;
- X₈ is T, A, K, R, Q, or E;
- X₉ is S, A, H, F, V, G, or T;
- X₁₀ is R, K, L, S, H, T, I, or A;
- X₁₁ is S, T, V, K, R, Y, M, or D;
- X₁₂ is S, R, or L; and
- X₁₃ is 1, H, or W.

7. The use according to claim 1 or 6 of mimotope decoys of the following formulas:
QCHTWSNRRSCL SEQ ID NO : 9
SCHAWSNRRTCR SEQ ID NO : 10
RCHAWSNRKSCV SEQ ID NO : 11
CSKWANRLVSIC SEQ ID NO : 12
CSKWHNRSKRHC SEQ ID NO : 13
ACTTWSNRSKCP SEQ ID NO : 14
ECTRWSNRSRCF SEQ ID NO : 15
CMKWSNRSSRWC SEQ ID NO : 16
KCGRWSNRSSCT SEQ ID NO : 17
CGRWFNRSDLHC SEQ ID NO : 18
ACHEWSNRSTCT SEQ ID NO : 19
KCSRWTNRHLCD SEQ ID NO : 20
KCTRWTNRHLCS SEQ ID NO : 21
KCTRWTNRAHCP SEQ ID NO : 22
QCSKWVNRSRCA SEQ ID NO : 23
NCQKWTNRRTCL SEQ ID NO : 24
QCGRWSNRSYCS SEQ ID NO : 25
TCHRWGNRTSCQ SEQ ID NO : 26
QCHRWANRISCS SEQ ID NO : 27
RCTQWTNRAYCP SEQ ID NO : 28
ACTQWSNRHMCG SEQ ID NO : 29
TCHPFSNRSTCT SEQ ID NO : 30

8. The use according to claim 7 of the mimotope decoy of the following formula:
SCHAWSNRRTCR SEQ ID NO : 10

9. The use according to claim 1 or 2 of the mimotope decoys of the following formulas
KCEPDDPWPQCI SEQ ID NO : 31
ACKRNHRWGACV SEQ ID NO : 32
ECGSHAWGRRCK SEQ ID NO : 33

10. The use according to claim 1 of mimotope decoys wherein X₈ represents the sequence:
(X₁₄)ₙ₁₄-X₁₅-X₁₆-H-X₁₇-W-G-X₁₈-(X₁₉)ₙ₁₉ **characterized in that**:
- X₁₄ represents K,
- X₁₅ represents R, ou G,
- X₁₆ represents N, ou S,
- X₁₇ represents R, ou A,
- X₁₈ represents A, ou R,
- X₁₉ represents R.

11. The use according to claim 1 of mimotope decoys wherein X₈ represents the sequence (X₁₄)ₙ₁₄-X₁₅-X₁₆-H-X₁₇-W-G-X₁₈-(X₁₉)ₙ₁₉ and has the following formula:
ECGSHAWGRRCK SEQ ID NO : 35

12. Pharmaceutical composition **characterized in that** it comprises mimotope decoys as defined in claims 1 to 11, in association with pharmaceutically acceptable vehicles.

13. Peptides of formula (I) as defined in claims 1 to 11.

14. Products comprising:
* at least one peptide of formula (I) according to claim 13,
* and at least one compound selected from extracted or recombinant factor VIII or other derivatives thereof from human or animal origin,
as combination product for simultaneous, separated or spread overtime use, in substitution therapy of factor VIII, in the framework of the treatment or prophylaxis of hemorrhagic accidents in patients with haemophilia A.

## Patentansprüche

1. Verwendung von mimotopen Ködern des Faktors VIII mit der folgenden Formel (I):
(X₁)n₁C-Xₐ-C-(X₂)ₙ₂
wobei:
- n₁ und n₂ unabhängig voneinander für 0 oder 1 stehen,
- X₁ und X₂ unabhängig voneinander für eine natürliche oder nicht natürliche Aminosäure stehen,
- Xₐ für eine der folgenden Sequenzen steht:
■ N P S I G D K N SEQ ID NR: 1; oder,
■ (X₃)n₃-X₄-X₅-G-K-T-X₆-L, wobei in der Sequenz
- n₃ für 0 oder 1 steht,
- X₃ für eine beliebige Aminosäure steht,
- X₄ für eine hydrophobe Aminosäure steht,
- X₅ für eine aromatische Aminosäure steht,
- X₆ für eine hydrophobe Aminosäure steht; oder
■ (X₇)-X₈-W-X₉-N-R-X₁₀-X₁₁-(X₁₂)ₙ₁₂-(X₁₃)ₙ₁₃, wobei in der Sequenz
- n₁₂ und n₁₃ unabhängig voneinander für 0 oder 1 stehen,
- X₇ bis X₁₃ für eine beliebige Aminosäure stehen,
- W gegebenenfalls ersetzt wird durch F oder
■ (X₁₄)ₙ₁₄-X₁₅-X₁₆-H-X₁₇-W-G-X₁₈-(X₁₉)ₙ₁₉, wobei in der Sequenz
- n₁₄ und n₁₉ unabhängig voneinander für 0 oder 1 stehen,
- X₁₄ bis X₁₈ für eine beliebige Aminosäure stehen, zur Herstellung von Medikamenten, die zur Vorbeugung oder Behandlung von Erkrankungen bestimmt sind, die mit dem Auftreten von Antikörpern gegen den endogenen Faktor VIII, oder gegen den exogenen Faktor VIII, oder rekombinante oder nicht rekombinante Derivate verbunden sind, die im Rahmen von Hämophilie A verabreicht werden.

2. Verwendung nach Anspruch 1 von mimotopen Ködern mit der Formel (I), wobei:
- X₁ ausgewählt ist aus Y, E, Q, S, R, A, K, N oder T,
- X₂ ausgewählt ist aus R, T, H, G, S, L, V, P, F, D, K, A, Q oder I.

3. Verwendung nach Anspruch 1 von mimotopen Ködern mit der Formel (I), wobei:
- Xₐ für Y C N P S I G D K N C R SEQ ID NR: 2 steht;

4. Verwendung nach Anspruch 1 von mimotopen Ködern, wobei Xₐ für die Sequenz (X₃)ₙ₃-X₄-X₅-G-K-T-X₆-L steht, **dadurch gekennzeichnet, dass:**
- X₃ ausgewählt ist aus I, Q, R, T oder K,
- X₄ ausgewählt ist aus V, T, L, I, M, F, W oder Y,
- X₅ ausgewählt ist aus F oder Y,
- X₆ ausgewählt ist aus A, M, Y, P, T oder V.

5. Verwendung nach Anspruch 1 oder 4 von mimotopen Ködern mit den folgenden Formeln:
E C I V Y G K T A L C T SEQ ID NR: 3
Q C P T F G K T M L C T SEQ ID NR: 4
S C R L F G K T Y L C H SEQ ID NR: 5
S C T V Y G K T P L C G SEQ ID NR: 6
R C K T F G K T T L C S SEQ ID NR: 7
R C T V Y G K T V L C L SEQ ID NR: 8.

6. Verwendung nach Anspruch 1 von mimotopen Ködern, wobei Xₐ für die folgende Sequenz steht:
X₇-X₈-W-X₉-N-R-X₁₀-X₁₁-(X₁₂)ₙ₁₂-(X₁₃)ₙ₁₃
**dadurch gekennzeichnet, dass:**
- X₇ ausgewählt ist aus H, S, T, M, Q oder G,
- X₈ ausgewählt ist aus T, A, K, R, Q oder E,
- X₉ ausgewählt ist aus S, A, H, F, V, G oder T,
- X₁₀ ausgewählt ist aus R, K, L, S, H, T, I oder A,
- X₁₁ ausgewählt ist aus S, T, V, K, R, Y, M oder D,
- X₁₂ ausgewählt ist aus S, R oder L,
- X₁₃ ausgewählt ist aus I, H oder W.

7. Verwendung nach Anspruch 1 oder 6 von mimotopen Ködern ausgewählt aus jenen mit den folgenden Formeln:
Q C H T W S N R R S C L SEQ ID NR: 9
S C H A W S N R R T C R SEQ ID NR: 10
R C H A W S N R K S C V SEQ ID NR: 11
C S K W A N R L V S I C SEQ ID NR: 12
C S K W H N R S K R H C SEQ ID NR: 13
A C T T W S N R S K C P SEQ ID NR: 14
E C T R W S N R S R C F SEQ ID NR: 15
C M K W S N R S S R W C SEQ ID NR: 16
K C G R W S N R S S C T SEQ ID NR: 17
C G R W F N R S D L H C SEQ ID NR: 18
A C H E W S N R S T C T SEQ ID NR: 19
K C S R W T N R H L C D SEQ ID NR: 20
K C T R W T N R H L C S SEQ ID NR: 21
K C T R W T N R A H C P SEQ ID NR: 22
Q C S K W V N R S R C A SEQ ID NR: 23
N C Q K W T N R R T C L SEQ ID NR: 24
Q C G R W S N R S Y C S SEQ ID NR: 25
T C H R W G N R T S C Q SEQ ID NR: 26
Q C H R W A N R I S C S SEQ ID NR: 27
R C T Q W T N R A Y C P SEQ ID NR: 28
A C T Q W S N R H M C G SEQ ID NR: 29
T C H P F S N R S T C T SEQ ID NR: 30.

8. Verwendung nach Anspruch 7 des mimotopen Köders mit der folgenden Formel:
S C H A W S N R R T C R SEQ ID NR: 10.

9. Verwendung nach Anspruch 1 oder 2 von mimotopen Ködern mit den folgenden Formeln:
K C E P D D P W P Q C I SEQ ID NR: 31
A C K R N H R W G A C V SEQ ID NR: 32
E C G S H A W G R R C K SEQ ID NR: 33.

10. Verwendung nach Anspruch 1 von mimotopen Ködern, wobei Xₐ für die Sequenz (X₁₄)ₙ₁₄-X₁₅-X₁₆-H-X₁₇-W-G-X₁₈-(X₁₉)ₙ₁₉ steht,
**dadurch gekennzeichnet, dass:**
- X₁₄ für K steht,
- X₁₅ für R oder G steht,
- X₁₆ für N oder S steht,
- X₁₇ für R oder A steht,
- X₁₈ für A oder R steht,
- X₁₉ für R steht.

11. Verwendung nach Anspruch 1 von mimotopen Ködern, wobei Xₐ für die Sequenz (X₁₄)ₙ₁₄-X₁₅-X₁₆-H-X₁₇-W-G-X₁₈-(X₁₉)ₙ₁₉ steht und die folgende Formel aufweist:
E C G S H A W G R R C K SEQ ID NR: 35.

12. Pharmazeutische Zusammensetzung, **dadurch gekennzeichnet, dass** sie Köder, wie in einem der Ansprüche 1 bis 11 definiert, in Assoziation mit pharmazeutisch verträglichen Vehikeln umfasst.

13. Peptide mit der Formel (I), wie in einem der Ansprüche 1 bis 11 definiert.

14. Produkte, umfassend:
* mindestens ein Peptid mit der Formel (I) nach Anspruch 13,
* und mindestens eine Verbindung, ausgewählt ist aus extrahiertem oder rekombinantem Faktor VIII oder anderen abgeleiteten Produkten humanen oder tierischen Ursprungs,
wie Kombinationsprodukten zur gleichzeitigen, getrennten oder zeitlich gestaffelten Verwendung bei der Faktor-VIII-Substitutionstherapie im Rahmen der Behandlung oder der Prophylaxe von schweren Blutungen bei Patienten, die an Hämophilie A leiden.


### SEQUENCE LISTING

<110> C.N.R.S.
<120> LEURRES PEPTIDIQUES POUR LA PREPARATION DE MEDICAMENTS DESTINES A LA PREVENTION OU AU TRAITEMENT DES PATHOLOGIES AUTO-IMMUNES, OU DES TROUBLES LIES A L'APPARITION D'ANTICORPS DIRIGES CONTRE DES PROTEINES EXOGENES
<130> IFB 01 AS CNR HUIT
<140> FR 01/13360
   <141> 2001-10-17
<160> 41
<170> PatentIn version 3.1
<210> 1
   <211> 8
   <212> PRT
   <213> séquence artificielle
<220>
   <223> leurre mimotopique du facteur VIII
<400> 1
   Asn Pro Ser Ile Gly Asp Lys Asn
   1               5
<210> 2
   <211> 12
   <212> PRT
   <213> séquence artificielle
<220>
   <223> leurre mimotopique du facteur VIII
<400> 2
   Tyr Cys Asn Pro Ser Ile Gly Asp Lys Asn Cys Arg
   1               5                   10
<210> 3
   <211> 12
   <212> PRT
   <213> séquence artificielle
<220>
   <223> leurre mimotopique du facteur VIII
<400> 3
   Glu Cys Ile Val Tyr Gly Lys Thr Ala Leu Cys Thr
   1 5 10
<210> 4
   <211> 12
   <212> PRT
   <213> séquence artificielle
<220>
   <223> leurre mimotopique du facteur VIII
<400> 4
   Gln Cys Pro Thr Phe Gly Lys Thr Met Leu Cys Thr
   1 5 10
<210> 5
   <211> 12
   <212> PRT
   <213> séquence artificielle
<220>
   <223> leurre mimotopique du facteur VIII
<400> 5
   Ser Cys Arg Leu Phe Gly Lys Thr Tyr Leu Cys His
   1 5 10
<210> 6
   <211> 12
   <212> PRT
   <213> séquence artificielle
<220>
   <223> leurre mimotopique du facteur VIII
<400> 6
   Ser Cys Thr Val Tyr Gly Lys Thr Pro Leu Cys Gly
   1 5 10
<210> 7
   <211> 12
   <212> PRT
   <213> séquence artificielle
<220>
   <223> leurre mimotopique du facteur VIII
<400> 7
   Arg Cys Lys Thr Phe Gly Lys Thr Thr Leu Cys Ser
   1 5 10
<210> 8
   <211> 12
   <212> PRT
   <213> séquence artificielle
<220>
   <223> leurre mimotopique du facteur VIII
<400> 8
   Arg Cys Thr Val Tyr Gly Lys Thr Val Leu Cys Leu
   1 5 10
<210> 9
   <211> 12
   <212> PRT
   <213> séquence artificielle
<220>
   <223> leurre mimotopique du facteur VIII
<400> 9
   Gln Cys His Thr Trp Ser Asn Arg Arg Ser Cys Leu
   1 5 10
<210> 10
   <211> 12
   <212> PRT
   <213> séquence artificielle
<220>
   <223> leurre mimotopique du facteur VIII
<400> 10
   Ser Cys His Ala Trp Ser Asn Arg Arg Thr Cys Arg
   1 5 10
<210> 11
   <211> 12
   <212> PRT
   <213> séquence artificielle
<220>
   <223> leurre mimotopique du facteur VIII
<400> 11
   Arg Cys His Ala Trp Ser Asn Arg Lys Ser Cys Val
   1               5                   10
<210> 12
   <211> 12
   <212> PRT
   <213> séquence artificielle
<220>
   <223> leurre mimotopique du facteur VIII
<400> 12
   Cys Ser Lys Trp Ala Asn Arg Leu Val Ser Ile Cys
   1               5                   10
<210> 13
   <211> 12
   <212> PRT
   <213> séquence artificielle
<220>
   <223> leurre mimotopique du facteur VIII
<400> 13
   Cys Ser Lys Trp His Asn Arg Ser Lys Arg His Cys
   1               5                   10
<210> 14
   <211> 12
   <212> PRT
   <213> séquence artificielle
<220>
   <223> leurre mimotopique du facteur VIII
<400> 14
   Ala Cys Thr Thr Trp Ser Asn Arg Ser Lys Cys Pro
   1               5                   10
<210> 15
   <211> 12
   <212> PRT
   <213> séquence artificielle
<220>
   <223> leurre mimotopique du facteur VIII
<400> 15
   Glu Cys Thr Arg Trp Ser Asn Arg Ser Arg Cys Phe
   1               5                   10
<210> 16
   <211> 12
   <212> PRT
   <213> séquence artificielle
<220>
   <223> leurre mimotopique du facteur VIII
<400> 16
   Cys Met Lys Trp Ser Asn Arg Ser Ser Arg Trp Cys
   1               5                   10
<210> 17
   <211> 12
   <212> PRT
   <213> séquence artificielle
<220>
   <223> leurre mimotopique du facteur VIII
<400> 17
   Lys Cys Gly Arg Trp Ser Asn Arg Ser Ser Cys Thr
   1               5                   10
<210> 18
   <211> 12
   <212> PRT
   <213> séquence artificielle
<220>
   <223> leurre mimotopique du facteur VIII
<400> 18
   Cys Gly Arg Trp Phe Asn Arg Ser Asp Leu His Cys
   1 5 10
<210> 19
   <211> 12
   <212> PRT
   <213> séquence artificielle
<220>
   <223> leurre mimotopique du facteur VIII
<400> 19
   Ala Cys His Glu Trp Ser Asn Arg Ser Thr Cys Thr
   1 5 10
<210> 20
   <211> 12
   <212> PRT
   <213> séquence artificielle
<220>
   <223> leurre mimotopique du facteur VIII
<400> 20
   Lys Cys Ser Arg Trp Thr Asn Arg His Leu Cys Asp
   1 5 10
<210> 21
   <211> 12
   <212> PRT
   <213> séquence artificielle
<220>
   <223> leurre mimotopique du facteur VIII
   <400> 21
   Lys Cys Thr Arg Trp Thr Asn Arg His Leu Cys Ser
   1 5 10
<210> 22
   <211> 12
   <212> PRT
   <213> séquence artificielle
<220>
   <223> leurre mimotopique du facteur VIII
<400> 22
   Lys Cys Thr Arg Trp Thr Asn Arg Ala His Cys Pro
   1 5 10
<210> 23
   <211> 12
   <212> PRT
   <213> séquence artificielle
<220>
   <223> leurre mimotopique du facteur VIII
<400> 23
   Gln Cys Ser Lys Trp Val Asn Arg Ser Arg Cys Ala
   1 5 10
<210> 24
   <211> 12
   <212> PRT
   <213> séquence artificielle
<220>
   <223> leurre mimotopique du facteur VIII
<400> 24
   Asn Cys Gln Lys Trp Thr Asn Arg Arg Thr Cys Leu
   1 5 10
<210> 25
   <211> 12
   <212> PRT
   <213> séquence artificielle
<220>
   <223> leurre mimotopique du facteur VIII
<400> 25
   Gln Cys Gly Arg Trp Ser Asn Arg Ser Tyr Cys Ser
   1 5 10
<210> 26
   <211> 12
   <212> PRT
   <213> séquence artificielle
<220>
   <223> leurre mimotopique du facteur VIII
<400> 26
   Thr Cys His Arg Trp Gly Asn Arg Thr Ser Cys Gln
   1 5 10
<210> 27
   <211> 12
   <212> PRT
   <213> séquence artificielle
<220>
   <223> leurre mimotopique du facteur VIII
<400> 27
   Gln Cys His Arg Trp Ala Asn Arg Ile Ser Cys Ser
   1 5 10
<210> 28
   <211> 12
   <212> PRT
   <213> séquence artificielle
<220>
   <223> leurre mimotopique du facteur VIII
<400> 28
   Arg Cys Thr Gln Trp Thr Asn Arg Ala Tyr Cys Pro
   1 5 10
<210> 29
   <211> 12
   <212> PRT
   <213> séquence artificielle
<220>
   <223> leurre mimotopique du facteur VIII
<400> 29
   Ala Cys Thr Gln Trp Ser Asn Arg His Met Cys Gly
   1 5 10
<210> 30
   <211> 12
   <212> PRT
   <213> séquence artificielle
<220>
   <223> leurre mimotopique du facteur VIII
<400> 30
   Thr Cys His Pro Phe Ser Asn Arg Ser Thr Cys Thr
   1 5 10
<210> 31
   <211> 12
   <212> PRT
   <213> séquence artificielle
<220>
   <223> leurre mimotopique du facteur VIII
<400> 31
   Lys Cys Glu Pro Asp Asp Pro Trp Pro Gln Cys Ile
   1 5 10
<210> 32
   <211> 12
   <212> PRT
   <213> séquence artificielle
<220>
   <223> leurre mimotopique du facteur VIII
<400> 32
   Ala Cys Lys Arg Asn His Arg Trp Gly Ala Cys Val
   1 5 10
<210> 33
   <211> 12
   <212> PRT
   <213> séquence artificielle
<220>
   <223> leurre mimotopique du facteur VIII
<400> 33
   Glu Cys Gly Ser His Ala Trp Gly Arg Arg Cys Lys
   1 5 10
<210> 34
   <211> 12
   <212> PRT
   <213> séquence artificielle
<220>
   <223> leurre mimotopique du facteur VIII
<400> 34
   Ala Cys Lys Arg Asn His Arg Trp Gly Ala Cys Val
   1 5 10
<210> 35
   <211> 12
   <212> PRT
   <213> séquence artificielle
<220>
   <223> leurre mimotopique du facteur VIII
<400> 35
   Glu Cys Gly Ser His Ala Trp Gly Arg Arg Cys Lys
   1 5 10
<210> 36
   <211> 15
   <212> PRT
   <213> séquence artificielle
<220>
   <223> variant épitopique du facteur VIII
<400> 36
   Asp Asp Asp Leu Thr Asp Ser Glu Met Asp Val Val Arg Phe Asp
   1 5 10 15
<210> 37
   <211> 15
   <212> PRT
   <213> séquence artificielle
<220>
   <223> variant épitopique du facteur VIII
<400> 37
   Asp Asp Asp Leu Thr Asp Ser Glu Met Asp Val Val Arg Phe Asp
   1 5 10 15
<210> 38
   <211> 15
   <212> PRT
   <213> séquence artificielle
<220>
   <223> variant épitopique du facteur VIII
<400> 38
   Asp Asp Asp Leu Thr Asp Ser Glu Met Asp Val Val Arg Tyr Asp
   1 5 10 15
<210> 39
   <211> 15
   <212> PRT
   <213> séquence artificielle
<220>
   <223> variant épitopique du facteur VIII
<400> 39
   Asp Asp Asp Leu Thr Asp Ser Glu Met Asp Val Val Arg Trp Asp
   1 5 10 15
<210> 40
   <211> 17
   <212> PRT
   <213> séquence artificielle
<220>
   <223> variant épitopique du facteur VIII
<400> 40
   Cys Asp Asp Asp Leu Thr Asp Ser Glu Met Asp Val Val Arg Phe Asp
   1 5 10 15
   Cys
<210> 41
   <211> 15
   <212> PRT
   <213> séquence artificielle
<220>
   <223> variant épitopique du facteur VIII
<400> 41
   Ala Ala Ala Leu Thr Asp Ser Glu Met Asp Val Val Arg Phe Ala
   1 5 10 15